# EUROPÄISCHE PATENTANMELDUNG

(11) **EP 1 076 340 A1**
(43) Veröffentlichungstag der Anmeldung: **14.02.2001**
(21) Anmeldenummer: 00250227.6
(22) Anmeldetag: 10.07.2000
(51) Int. Cl.: G21F 5/015

(54) **Sterilisierbarer Strahlenschutzbehälter und Verfahren zur Sterilisierung radioaktiver Strahlenquellen**

(30) Priorität: 11.08.1999 DE 19938027
(71) Anmelder: Bebig Isotopentechnik und Umweltdiagnostik GmbH, 13125 Berlin (DE)
(72) Erfinder: Danlewski, Harald Dr., verstorben (DE)
(74) Vertreter: Hengelhaupt, Jürgen D., Dipl.-Ing.

(57) **Zusammenfassung**

Die Erfindung beschreibt einen sterilisierbaren Strahlenschutzbehälter für radioaktive Strahlenquellen sowie ein Verfahren zur Sterilisierung derartiger Strahlenquellen. Der Strahlenschutzbehälter besteht aus einem Grundkörper 1 und einem Deckel 2, welche einen Hohlraum 5 zur Aufnahme der Strahlenquellen bilden und im Grundkörper 1 sowie im Deckel 2 jeweils mindestens eine Öffnung 3 zum Durchströmen des Hohlraumes 5 mit einem Sterilisierungsmedium angeordnet sind. Das Verfahren zur Sterilisierung der radioaktiven Strahlenquellen basiert darauf, daß die Sterilisation direkt in einem zum Transport geeigneten Strahlenschutzbehälter erfolgt derart, daß die Strahlenquellen in den Strahlenschutzbehälter eingebracht werden, der Strahlenschutzbehälter mit einem Deckel verschlossen wird, der verschlossene Strahlenschutzbehälter in eine medizinische Verpackung eingebracht und die medizinische Verpackung verschlossen wird und die medizinische Verpackung mit dem darin enthaltenen Strahlenschutzbehälter in einem Sterilisator mittels gasförmiger Sterilisationsmedien sterilisiert wird, indem die gasförmigen Sterilisationsmedien die medizinische Verpackung durchdringen und den Strahlenschutzbehälter mit den Strahlenquellen durch dafür vorgesehene Öffnungen im Strahlenschutzbehälter durchströmen.

## Beschreibung

Die Erfindung betrifft sterilisierbare Strahlenschutzbehälter, insbesondere für umschlossene radioaktive Strahlenquellen sowie ein Verfahren zur Sterilisierung derartiger Strahlenquellen.

Die Erfindung beschreibt zwei Varianten einer Behälterbauart, die gleichzeitig für den Transport, die Lagerung und die Sterilisierung bei Gewährleistung des notwendigen Strahlenschutzes von umschlossenen radioaktiven Strahlenquellen geeignet sind. Die erfindungsgemäße Behälterbauart besteht aus einem Außenbehälter, welcher einerseits die radioaktive Strahlung ausreichend abschwächt und andererseits den Eintritt von Wasserdampf oder Gas gestattet, um dadurch die Strahlenquellen sterilisieren zu können. Dieser Außenbehälter dient zur Aufnahme geeigneter handelsüblicher Innenbehälter, welche die radioaktiven umschlossenen Strahlenquellen beherbergen.

Umschlossene radioaktive Quellen werden beispielsweise in der Onkologie zur Behandlung von Prostatakrebs oder in der Kardiologie zur Vermeidung von Restenosen nach einer perkutanen Ballondilitation eingesetzt. Im Fall der Prostatakrebabehandlung werden die Strahlenquellen dauerhaft im Menschen implantiert. Aus diesen Gründen ist eine Sterilisierung der Strahlenquellen vor der Anwendung notwendig. Bei der Handhabung von radioaktiven Materialien sind besondere Vorsichtsmaßnahmen zum Schutz des Personals, welche die Sterilisation durchzuführen hat, erforderlich.

Bisher werden gemäß dem bekannten Stand der Technik für Transport und Sterilisation von umschlossenen radioaktiven Strahlenquellen getrennte unterschiedliche Behälter genutzt. Für den Transport werden die Strahlenquellen beispielsweise in einer Bleiflasche abgefüllt, um vor der Sterilisation in eine Sterilisationsbox umgefüllt zu werden.

Ein spezieller Transport- und Lagerbehälter, welcher jedoch nicht für die Sterilisation geeignet ist, ist in der DE 198 36 188.2 beschrieben.

Der Erfindung liegt die Aufgabe zugrunde, einen Strahlenschutzbehälter zu schaffen, in welchem die Strahlenquellen sowohl sterilisiert als auch aufbewahrt und transportiert werden können. Dabei soll der Strahlenschutzbehälter einfach zu handhaben und preiswert herstellbar sein und jederzeit einen ausreichenden Strahlenschutz bieten.
Es ist weiterhin Aufgabe der Erfindung, ein Verfahren anzugeben, mit welchem die Sterilisierung von Strahlenquellen sicher, effektiv und nutzerfreundlich möglich ist.

Diese Aufgabe wird erfindungsgemäß gelöst durch die Merkmale im kennzeichnenden Teil der Ansprüche 1 und 9 in Verbindung mit den Merkmalen in den Oberbegriffen. Zweckmäßige Ausgestaltung der Erfindung sind in den Unteransprüchen enthalten.

Ein besonderer Vorteil der Erfindung besteht in der Multifunktionalität des Strahlenschutzbehälters als Sterilisierungsbehälter, Lagerbehälter und Transportbehälter, welche dadurch erreicht wird, daß der Strahlenschutzbehälter aus einem Grundkörper und einem Deckel besteht, welche einen Hohlraum zur Aufnahme der Strahlenquellen bilden und im Grundkörper sowie im Deckels jeweils mindestens eine Öffnung zum Durchströmen des Hohlraumes mit einem Sterilisierungsmedium angeordnet sind.
Bei der vorliegenden Erfindung können die Strahlenquellen in einer beliebig geeigneten Form von Innenbehälter, beispielsweise in Form eines Magazins abgepackt, und anschließend in den äußeren Behälter eingeführt werden. Die Erfindung erlaubt aufgrund der vorhandenen Öffnungen den Eintritt von z.B. Wasserdampf und gewährleistet somit die Sterilisation. Anderseits führt die geeignete Materialverwendung zu einer Abschirmung von radioaktiver Strahlung. Die Öffnungen sind so konstruiert, daß kein direkter Strahlendurchgang möglich ist.

Gemäß einer Modifikation der Erfindung ist es möglich, die Öffnungen in dem Strahlenschutzbehälter mittels Sterilisationsschutzmaterial, z.B. Sterilisationspapier, zu verschließen und hierdurch Verpackungs- und Versandmaterial einzusparen.

Ein weiterer Vorteil der Erfindung besteht darin, daß die Sterilisation direkt in einem zum Transport geeigneten Strahlenschutzbehälter erfolgt derart, daß die Strahlenquellen in den Strahlenschutzbehälter eingebracht werden, der Strahlenschutzbehälter verschlossen wird, der verschlossene Strahlenschutzbehälter in eine medizinische Verpackung eingebracht und die medizinische Verpackung verschlossen wird und die medizinische Verpackung mit dem darin enthaltenen Strahlenschutzbehälter in einem Sterilisator mittels gasförmiger Sterilisationsmedien sterilisiert wird, indem die gasförmigen Sterilisationsmedien die medizinische Verpackung durchdringen und den Strahlenschutzbehälter mit den Strahlenquellen durch dafür vorgesehene Öffnungen im Strahlenschutzbehälter durchströmen.

Die vorliegende Erfindung gestattet das vollautomatische Verpacken der Strahlenquellen in einem Behälter, der im geschlossenen Zustand einen optimalen Strahlenschutz gewährleistet. Im geschlossenen Zustand kann gefahrlos der Behälter per Hand angefasst werden. Die allgemeine Konstruktionsart des Außenbehälters erlaubt auch im geschlossenen Zustand das Einströmen von Wasserdampf oder Gas (Ethylenoxid, Formaldehyd). Die ausreichende Abschirmung der radioaktiven Strahlung wird durch die richtige Dimensionierung der Behälterwandstärke und die Verwendung geeigneter Behältermaterialien erreicht. Die Dimensionierung und das Material kann nach üblichen, dem Fachmann gut bekannten Methoden optimal gewählt werden. Alle erfindungsgemäß eingesetzten Werkstoffe sind kommerziell erhältlich, z.B. Edelstahl. Der Wasserdampf oder das Gas kann durch spezielle Öffnungen eindringen, die aufgrund ihrer geometrischen Anordnung den Austritt von radioaktiven Teilchenstrahlen (Gamma-, Beta-, Alphateilchen) fast vollständig verhindern. Die Herstellung des Außenbehälters ist mit den gängigen Metallbearbeitungamaschinen in einfacher Weise möglich und kann leicht den jeweiligen Abmessungen des Innenbehälters angepaßt werden. Als Innenbehälter können nahezu beliebige halboffene Behälter verwendet werden. Auch nach dem Öffnen des Deckels des Außenbehälters ist noch ein seitlicher Strahlenschutz gewährleistet. Die Entnahme des Innenbehälters kann beispielsweise mittels einer Pinzette/Zange erfolgen.

Die Erfindung soll nachstehend anhand von zumindest teilweise in den Figuren dargestellten Ausführungsbeispielen näher erläutert werden. Es zeigen
- Fig. 1: einen sterilisierbaren Strahlenschutzbehälter für in einem Innenbehälter angeordnete Strahlenquellen
- Fig. 2: einen sterilisierbaren Strahlenschutzbehälter für in sieben Innenbehältern angeordnete Strahlenquellen

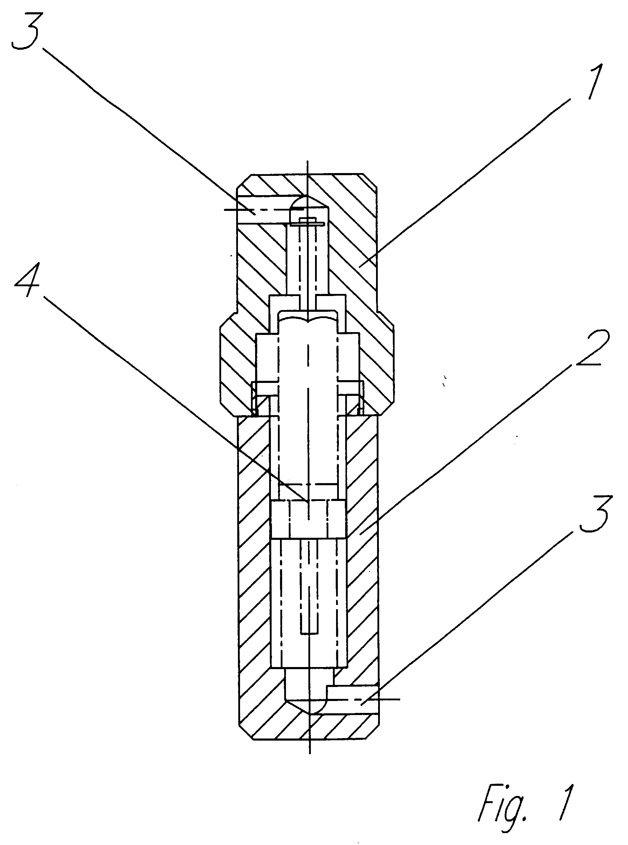

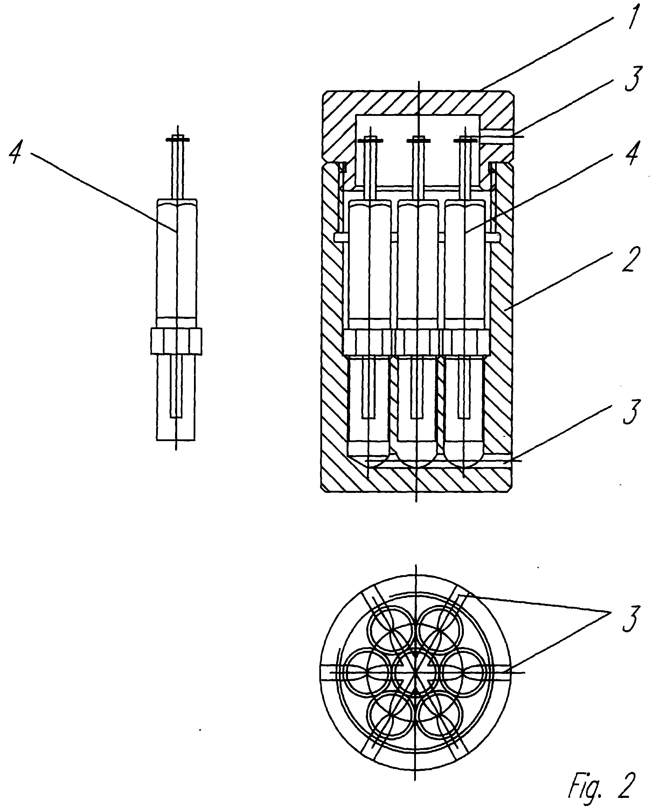

Die in den Fig. 1 und 2 dargestellten Strahlenschutzbehälter zeigen beispielhaft zwei Ausführungsformen für erfindungsgemäße Außenbehälter. Diese Ausführungsformen wurden in ihren Abmessungen auf kommerziell erhältliche Innenbehälter und den sich darin befindenden z. Bsp. IsoSeed I-125 Strahlenquellen angepaßt.

Wie aus Fig. 1 zu ersehen ist, besteht der erfindungsgemäße Strahlenschutzbehälter aus einem Grundkörper 1 und einem darauf aufgeschraubten Deckel 2. Von dem Grundkörper 1 und dem Deckel 2 umschlossen wird ein Hohlraum 5 gebildet, in welchem in vorliegenden Ausführungsbeispiel ein Innenbehälter 4, in welchem sich die Strahlenquellen geordnet eingebracht befinden, angeordnet ist. Am Boden des Grundkörper 1 und im Kopfbereich des Deckels 2 ist jeweils eine Öffnung 3 angeordnet, durch welche das Sterilisierungsmedium den Hohlraum 5 durchströmen und somit die in Innenbehälter 4 enthaltenen Strahlungsquellen sterilisieren kann.

Der Strahlenschutzbehälter ist derart ausgebildet, daß der Grundkörper 1 mit seiner Mantelwandung die Strahlenquellen auch bei abgenommenem Deckel 2 umschließt, wobei der Grundkörper 1 und der Deckel 2 aus Edelstahl bestehen.
Ebenso ist es möglich, daß der Grundkörper 1 und/oder der Deckel 2 einen Sandwichaufbau aufweisen mit einer Schichtfolge Edelstahl-Blei-Edelstahl.

In Fig. 2 ist eine Ausführungsvariante dargestellt, bei welcher der Hohlraum 5 zur Aufnahme von mehreren Innenbehältern 4 ausgebildet ist, und der Hohlraum 5 zur Aufnahme von insgesamt sieben Innenbehältern 4 dient, wobei jedem Innenbehälter 4 eine Öffnung 3 zugeordnet ist. Eine modifizierte Ausführungsform der Strahlenschutzbehälter basiert darauf, daß die Öffnungen 3 mit einem Sterilisationsschutzmaterial verschlossen sind, wobei das Sterilisationsschutzmaterial Sterilisationspapier ist.

Der Vorgang der Sterilisierung verläuft so, die Sterilisation direkt in einem zum Transport geeigneten Strahlenschutzbehälter erfolgt derart, daß die Strahlenquellen in den Strahlenschutzbehälter eingebracht werden, der Strahlenschutzbehälter beispielsweise durch einen Deckel verschlossen wird, der verschlossene Strahlenschutzbehälter in eine medizinische Verpackung eingebracht und die medizinische Verpackung verschlossen wird und die medizinische Verpackung mit dem darin enthaltenen Strahlenschutzbehälter in einem Sterilisator mittels gasförmiger Sterilisationsmedien sterilisiert wird, indem die gasförmigen Sterilisationsmedien die medizinische Verpackung durchdringen und den Strahlenschutzbehälter mit den Strahlenquellen durch dafür vorgesehene Öffnungen im Strahlenschutzbehälter durchströmen. Das Sterilisationsmedium ist im vorliegenden Ausführungsbeispiel feuchte Hitze bzw. Wasserdampf, jedoch sind ebenso Gase wie Ethylenoxid oder Formaldehyd anwendbar.
Als medizinische Verpackung wird ein Sterilisationsschutzmaterial, im vorliegenden Anwendungsbeispiel eine Verpackung mit Sterilisationspapier, verwendet. Das Sterilisationspapier hat die Eigenschaft, daß sich die Poren des Papiers während dem Sterilisationsvorgang öffnen und das Sterilisationsmedium durchlassen, während nach Abschluß der Sterilisation die wieder geschlossenen Poren einen hermetischen Abschluß gegen Eindringen von Keimen gewährleisten.

Die Erfindung ist nicht beschränkt auf die hier dargestellten Ausführungsbeispiele. Vielmehr ist es möglich, durch Kombination und Modifikation der genannten Mittel und Merkmale weitere Ausführungsvarianten zu realisieren, ohne den Rahmen der Erfindung zu verlassen.

## Patentansprüche

1. Sterilisierbarer Strahlenschutzbehälter für radioaktiver Strahlenquellen,
dadurch gekennzeichnet, daß
der Strahlenschutzbehälter aus einem Grundkörper (1) und einem Deckel (2) besteht, welche einen Hohlraum (5) zur Aufnahme der Strahlenquellen bilden und im Grundkörper (1) sowie im Deckel (2) jeweils mindestens eine Öffnung (3) zum Durchströmen des Hohlraumes (5) mit einem Sterilisierungsmedium angeordnet ist.

2. Sterilisierbarer Strahlenschutzbehälter nach Anspruch 1,
dadurch gekennzeichnet, daß der Hohlraum (5) zur Aufnahme von einem Innenbehälter (4) oder mehreren Innenbehältern (4) ausgebildet ist und die Strahlenquellen in Innenbehältern (4) in dem Hohlraum (5) angeordnet sind.

3. Sterilisierbarer Strahlenschutzbehälter nach Anspruch 1,
dadurch gekennzeichnet, daß der Grundkörper (1) mit seiner Mantelwandung die Strahlenquellen auch bei abgenommenem Deckel (2) umschließt.

4. Sterilisierbarer Strahlenschutzbehälter nach Anspruch 1,
dadurch gekennzeichnet, daß der Grundkörper (1) und der Deckel (2) aus Edelstahl bestehen oder daß der Grundkörper (1) und/oder der Deckel (2) einen Sandwichaufbau aufweisen mit einer Schichtfolge Edelstahl-Blei-Edelstahl.

5. Sterilisierbarer Strahlenschutzbehälter nach Anspruch 2,
dadurch gekennzeichnet, daß der Hohlraum (5) zur Aufnahme von insgesamt sieben Innenbehältern (4) ausgebildet ist und jedem Innenbehälter (4) eine Öffnung (3) zugeordnet ist.

6. Sterilisierbarer Strahlenschutzbehälter nach Anspruch 1,
dadurch gekennzeichnet, daß die Öffnungen (3) derart angeordnet sind, daß kein direkter Strahlendurchgang möglich ist und der Deckel (2) auf den Grundkörper (1) über ein Gewinde aufschraubbar ist.

7. Sterilisierbarer Strahlenschutzbehälter nach Anspruch 1,
dadurch gekennzeichnet, daß die Öffnungen (3) mit einem Sterilisationsschutzmaterial verschlossen sind.

8. Sterilisierbarer Strahlenschutzbehälter nach Anspruch 7,
dadurch gekennzeichnet, daß das Sterilisationsschutzmaterial Sterilisationspapier ist.

9. Verfahren zur Sterilisierung von radioaktiven Strahlenquellen,
dadurch gekennzeichnet, daß die Sterilisation direkt in einem zum Transport geeigneten Strahlenschutzbehälter erfolgt derart, daß
- die Strahlenquellen in den Strahlenschutzbehälter eingebracht werden,
- der Strahlenschutzbehälter verschlossen wird,
- der verschlossene Strahlenschutzbehälter in eine medizinische Verpackung eingebracht und die medizinische Verpackung verschlossen wird,
- die medizinische Verpackung mit dem darin enthaltenen Strahlenschutzbehälter in einem Sterilisator mittels gasförmiger Sterilisationsmedien sterilisiert wird, indem die gasförmigen Sterilisationsmedien die medizinische Verpackung durchdringen und den Strahlenschutzbehälter mit den Strahlenquellen durch dafür vorgesehene Öffnungen im Strahlenschutzbehälter durchströmen.

10. Verfahren nach Anspruch 9,
dadurch gekennzeichnet, daß die Sterilisationsmedien Wasserdampf oder feuchte Hitze oder Ethylenoxid oder Formaldeyd sind.

11. Verfahren nach Anspruch 9,
dadurch gekennzeichnet, daß die Strahlenquellen geordnet in den Strahlenschutzbehälter eingebracht werden und die Öffnungen im Strahlenschutzbehälter derart angeordnet sind, daß kein direkter Strahlenaustritt möglich ist.
